# EUROPEAN PATENT SPECIFICATION

(11) **EP 2 272 488 B1**
(45) Date of publication and mention of the grant of the patent: **14.12.2011**
(21) Application number: 09729284.1
(22) Date of filing: 09.04.2009
(51) Int. Cl.: A61K 8/14, A61K 8/46, A61K 8/60, A61K 9/127, A61K 47/20, A61K 47/26, A61Q 19/00

(54) **VESICLE AND COSMETIC CONTAINING THE SAME**
VESIKEL UND KOSMETIK DIESES ENTHALTEND
VÉSICULE ET COSMÉTIQUE CONTENANT CETTE VÉSICULE

(30) Priority: 09.04.2008 JP 2008101721
(43) Date of publication of application: 12.01.2011
(73) Proprietor: Shiseido Company, Ltd., Chuo-ku Tokyo 104-8010 (JP)
(72) Inventor: TAKAHASHI, Akiko, Yokohama-shi Kanagawa 224-8558 (JP); OKAMOTO, Tohru, Yokohama-shi Kanagawa 224-8558 (JP)
(74) Representative: Lippert, Stachow & Partner
(86) International application number: PCT/JP2009/057282
(87) International publication number: WO 2009/125816

(56) References cited:
- WO-A1-2004/004676
- JP-A- 3 074 323
- JP-A- 5 004 037
- JP-A- 64 000 014
- JP-A- 2001 097 811
- JP-A- 2004 352 643
- JP-A- 2008 074 780
- JP-B2- 3 126 193

## Description

### RELATED APPLICATIONS

This application claims the priority of Japanese Patent Application No. 2008-101721 filed on April 9, 2008, which is incorporated herein by reference.

### FIELD OF THE INVENTION

The present invention relates to a vesicle and particularly to improving stability of the vesicle in cosmetics.

### BACKGROUND OF THE INVENTION

A technique of encapsulating an active ingredient such as a medicinal agent into microcapsules, which are externally and internally applied to a living body is advantageous because metabolism of the content within the capsules is suppressed and the efficacy of the acitive ingredient is maintained for a long time. Because of the advantage, the technique has been investigated with a view to practical use not only in the fields of medicine and food but also the fields of cosmetics.

As a microencapsulation technique in the cosmetic field, use of liposomes and vesicles (closed endoplasmic reticulum formed of bimolecular lipid membrane) containing a phospholipid such as lecithin as a membrane component has been conventionally known. However, these components were not sufficient to put in practical use in view of properties such as thermal stability, long term stability of vesicle structure and cost performance.

To deal with the problem, it has been reported that when a vesicle (closed endoplasmic reticulum formed of a bilayer lipid membrane) using a sucrose fatty acid diester as a membrane component is produced in the presence of an ionic surfactant, an extremely stable vesicle structure can be easily obtained (Patent Document 1). The technique provides excellent thermal stability Tc (gel-liquid crystal transition temperature) is higher than that of the phospholipid, with a high rate of encapsulating the active ingredient in a water dispersion system particularly at a temperature of Tc or less.

Patent Literature 1 : Japanese Patent No. 3126193

### DISCLOSURE OF THE INVENTION

### PROBLEM TO BE SOLVED BY THE INVENTION

However, a sucrose fatty acid diester used as a raw material and obtained in a large scale of industrial production contains a large amount of impurities such as a monoester and a triester. Therefore, to obtain a stable vesicle maintaining a predetermined particle size and encapsulation rate by use of the sucrose fatty acid diester, it has been necessary to remove impurities via a complicated raw-material purification step. In short, although it is apparent that a sucrose fatty acid diester forms a stable vesicle, it is difficult to supply it constantly as a pure compound and a problem remains in the productivity as a product. Furthermore, the dispersion stability of the vesicle in a solvent containing ethanol and the like is low. Practical use of the vesicle in lotion and the like is still insufficient and unsatisfactory.

The present invention was made in view of the problems in the prior art and is directed to provide a vesicle having excellent dispersion stability.

### MEANS TO SOLVE THE PROBLEM

To attain the object, the present inventors have conducted intensive studies. As a result, they found that a highly stable vesicle containing a sucrose fatty acid diester as a main membrane component is formed by adding an appropriate amount of predetermined ionic surfactant, and further that the vesicle maintains excellent stability even in a solvent containing ethanol and the like. Based on the finding, the present invention was accomplished.

More specifically, a vesicle according to the present invention is characterized in that it comprises a sucrose fatty acid diester and an acyl methyl taurate being sodium stearoyl methyl taurate or sodium lauroyl methyl taurate in an amount of 5 to 30 wt% relative to the diester, in which the sucrose fatty acid diester is contained as a main component of membrane.

Furthermore, the content of impurities in the sucrose fatty acid diester is up to 60%. Furthermore, a cosmetic according to the present invention is characterized in that it comprises the vesicle.

Furthermore, a lotion according to the present invention is characterized in that the vesicle is dispersed in a solvent containing alcohol.

Furthermore, a method for producing a vesicle according to the present invention is characterized in that it comprises:
(I) dissolving a sucrose fatty acid diester in a water-soluble solvent under heating; and
(II) mixing a solution obtained in (I) and an aqueous solvent containing sodium stearoyl methyl taurate or sodium lauroyl methyl taurate.

### EFFECT OF THE INVENTION

According to the present invention, it is possible to obtain an extremely stable vesicle containing a sucrose fatty acid diester as a main membrane component. The vesicle added to a cosmetic exhibits excellent moisturizing effect and usability. Furthermore, the vesicle can be used in an alcohol-base lotion to which a conventional vesicle was difficult to add. Moreover, the vesicle is highly stable as a carrier (DDS base) for an acitive ingredient etc., and can be applied to both water soluble and lipid-soluble acitive ingredient.

### BRIEF DESCRIPTION OF THE DRAWING

Fig. 1 is an illustration showing a test method regarding the moisture retention effect skin moisturizing effect of a lotion containing a vesicle according to the present invention.

Fig. 2 is a graph showing the water content of the horny layer when a lotion containing a vesicle according to the present invention is applied.

Fig. 3 is a graph showing evaluation on usability of a lotion containing a vesicle according to the present invention.

### DESCRIPTION OF THE PREFERRED EMBODIMENT

The constitution of the present invention will be more specifically described below.

A vesicle according to the present invention comprises a sucrose fatty acid diester and an acyl methyl taurate, namely sodium stearoylmethyl taurate or sodium lauroyl methyl taurate.

The fatty acids of the sucrose fatty acid diester are saturated or unsaturated linea or branched fatty acids having 12 to 22 carbon atoms. These two fatty acids may be different. Note that, it is extremely difficult to disperse the sucrose fatty acid diester in water even at a temperature of Tc (gel-liquid crystal transition temperature) or more. Therefore, it is necessary to add an acyl methyl taurate serving as a vesicle-forming agent in an amount of 5 to 30 wt% relative to the diester, preferably 5 to 15 wt%, and further preferably, 5 to 10 wt%. When the content of the acyl methyl taurate is less than 5 wt% relative to the diester, the stability of the vesicle tends to decrease. On the other hand, the content exceeding 30 wt% is not preferable, since the acyl methyl taurate may precipitate as a crystal.

Examples of an acyl methyl taurate may additionally include a coconut fatty acid methyl taurate, a palm kernel fatty acid methyl taurate, a hydrogenated palm kernel fatty acid methyl taurate, a tallow fatty acid methyl taurate, a hydrogenated tallow fatty acid methyl taurate, a caproyl methyl taurate, a lauroyl methyl taurate, a myristoyl methyl taurate, a palmitoyl methyl taurate, a stearoyl methyl taurate, an oleoyl methyl taurate and the like.

Furthermore, examples of a preferable counter ion include Na, K, triethanolamine, ammonia and the like. In the present invention, particularly sodium lauroyl methyl taurate is preferably used.

Furthermore, a sucrose fatty acid diester may contain other types of sucrose fatty acid esters, for example, a monoester and a triester as impurities. Specifically, if a sucrose fatty acid diester is contained in an amount of 40% or more in a raw material, a stable vesicle can be produced. More specifically, if the content of impurities in a raw material is up to 60%, the raw material can be directly subjected to vesicle formation. Usually, about 50 to 60% of impurities remains in a commercially available sucrose fatty acid diester. Therefore, in producing a vesicle using a conventional sucrose fatty acid diester as a membrane component, a complicated treatment is required for removing these impurities to highly purify the diester. On the other hand, according to a method for producing a vesicle of the present invention, even if impurities are contained within the range, a vesicle can be formed without affecting the stability.

A vesicle according to the present invention can be easily produced in the form of a vesicle dispersion solution by dissolving the sucrose fatty acid diester in a water soluble solvent (e.g., ethanol and dipropylene glycol) heated, adding this to an aqueous solvent containing an acyl methyl taurate and heated to about the same temperature as above, mechanically mixing and stirring them followed by cooling.

The heating temperature of the solvent in a production process is preferably, Tc of a sucrose fatty acid diester, i.e., 50°C or more, more specifically, 50 to 100°C, and further preferably, 60 to 80°C. When a sucrose fatty acid diester is mixed with an aqueous solvent while stirring under the heating, the diester whose flowability has been increased by heating is dispersed in the form of particles uniform in size in a water phase.

In the production method, mechanical stirring/mixing means is not limited. Examples thereof that are used include a homo mixer, a nanomizer, a colloidal mill, a microfluitizer, a supersonic emulsifier and the like. Furthermore, if conditions for stirring/mixing by these means are adjusted, the particle-size of the vesicle to be formed can be controlled. More specifically, it is considered that the finer the particles of a sucrose fatty acid diester dispersed in a water phase by increasing stirring/mixing conditions to a high level, the smaller the particle size of the vesicles to be formed.

Depending on the volume of solution mixture and the components to be added, stirring/mixing, for example, by a homo mixer at 3500 to 9000 rpm for about 1 to 5 minutes, can result in vesicles having an average particle size of about 50 to 200 nm.

The water soluble solvent in the production method refers to a solvent freely miscible with water and slightly having lipophilicity. Examples of such water soluble solvent includes ethanol, isopropyl alcohol, ethylene glycol, propylene glycol, 1,3-butylene glycol, hexylene glycol, diethylene glycol, dipropylene glycol, polyethylene glycol, polypropylene glycol, glycerin, diglycerin, polyglycerin and the like. These may be added singly or in combination with two or more types. The content of the water soluble solvent relative to the total amount of components of the production system is preferably 1 to 20 wt%. Furthermore, the sucrose fatty acid diester to be dissolved in the water soluble solvent is preferably contained in an amount of 0.05 to 5 wt% relative to the total amount of components of the production system, and more preferably 0.1 to 1 wt%.

Furthermore, the aqueous solvent for use in production of the present invention represents water or an aqueous solution of a water soluble component and corresponds to a water phase component in a system.

The interior within the lipid membrane of a vesicle formed in an aqueous solvent in accordance with the vesicle production method is filled with an aqueous solvent serving as a dispersion medium of the vesicle. More specifically, if desired, a compound such as a medicinal agent is previously dispersed or dissolved in an aqueous solvent when a vesicle is produced, the compound can be allowed to be present within the vesicle. Furthermore, if a compound is added to an aqueous solvent having the vesicle dispersed therein or if a vesicle isolated is dispersed in a compound solution prepared in a desired concentration, the compound can be introduced into the vesicle.

Furthermore, if the compound to be introduced is soluble in lipid, the compound is dissolved in a water-soluble solvent together with a sucrose fatty acid diester when the vesicle is produced. In this manner, the compound can be introduced into a lipid layer formed of bilayer membrane forming a vesicle.

Examples of the substance to be introduced into a vesicle include a medicinal agent (a substance to be applied for a medicinal effect including not only substances not present in the nature but also physiologically active substances present in a living body in different amounts) and a label (a substance which is administered for diagnosis, etc., and capable of generating a detectable signal). Accordingly, such a substance can be used as an effective carrier particularly in a drug delivery system (DDS).

As the compound that can be introduced into a vesicle, all compounds can be used as long as they are not, for example, denatured by interaction with a vesicle component. Note that vesicles containing various types of compounds can be used not only in the medical field but also in the fields of cosmetics and food.

Examples of the applicable compound include enzymes such as cytochrome P450, cytochrome P450 reductase and SOD; gene related substances such as DNA and RNA; physiologically active substances such as an interleukin, interferon-α, -β, -γ, TPA, a lymphotoxin and ceruletide; and prostaglandins. Other than these, examples thereof include a relief of headache, an antipyretic drug, an anti-inflammatory drug (e.g., ergot alkaloid, a morphine, pentazocine, aspirin, ibuprofen, indomethacin, acetaminophen), a medicine for a mind/nervous system disease (e.g., diazepam, ethosuximide, phenytoin, carbamazepine, phenobarbital, sodium valproate, levodopa, trihexyphenidyl hydrochloride, amantadine hydrochloride, imipramine hydrochloride, amitriptyline hydrochloride, chlordiazepoxide, chlorpromazine hydrochloride, haloperidol), a medicine for a heart/vascular disease (e.g., digoxin, dobutamine, isoproterenol, epinephrine, propranolol, nifedipine, quinidine, hydrazine, hydrochlorothiazide, reserpine, prazosin, guanethidine, furosemide, chlorthalidone, spironolactone), an anti-allergy/anti-asthmatic drug (e.g., diphenhydramine, chlorpheniramine maleate, sodium cromoglycate, salbutamol sulfate, ipratropium bromide), an antirheumatic/antarthritic drug (e.g., phenylbutazone, D-penicillamine, an immunosuppressive drug, allopurinol, sulfinpyrazone, naproxen), an antibacterial agent (e.g., a penicillin-base antibacterial agent, a cephalosporin-base antibacterial agent, gentamicin, minocycline, erythromycin, rifampicin, isoniazid, kanamycin, griseofulvin, nystatin), diphtheria antitoxin, antivenin serum and a vaccine, etc., an antiparasitic/antiprotozoal agent (e.g., metronidazole, dehydroemetine, suramin sodium, niclosamide), an anti-tumor/antileukemic drug (e.g., busulfan, cyclophosphamide, bleomycin, fluorouracil, methotrexate), an anti-lipemic/anti-diabetic agent (e.g., clofibrate, tolbutamide, chlorpropamide), a medicine for a blood disease (e.g., fibrinogen, factor VIII, heparin, cyanocobalamin), a medicine for a digestive organ (e.g., acrinol, diastase, pancreatin), a hormone-related medicine (e.g., hydrocortisone, prednisolone, dexamethasone, methyltestosterone, estrogen, insulin, levothyroxine), a vitamin (e.g., vitamin A, active-form vitamin B I, vitamin C, vitamin E, pantothenic acid), a nutritional alteration medicine (e.g., calcium aspartate, isoleucine, ferrous orotate), an agents for external use, and a whitening agent (e.g., hydroquinone, arbutin).

Examples of the label include an X-ray contrast agent (e.g., metrizamide, metrizoic acid), a radioactive or non-radioactive (stable) isotope preparation and other CT preparations.

Particularly, a water soluble medicinal agent such as arbutin, tranexamic acid (TA) and a lipid-form medicinal agent such as retinol, Thujae Semen, linoleic acid, linolenic acid and ceramide can be preferably introduced.

When the vesicle according to the present invention is incorporated in a known cosmetic base, a cosmetic excellent in moisture retention effect skin moisturizing effect and usability can be obtained.

Alternatively, in producing the vesicle dispersion solution, if a component that can be used in general cosmetics, such as a powder component, an oily component, various surfactants, a water-soluble polymer, a UV absorbent, a lower alcohol, an organic amine, a sequestering agent, an antioxidant, a moisturizing agent, polyol, a monosaccharide, an oligosaccharide, an amino acid, a polymer emulsion, a pH regulator, a vitamin, a preservative, a whitening agent, an anti-inflammatory agent, a blood circulation accelerant, an extract, an activator, an antiseborrheic agent and a fragrance, is appropriately added as long as it does not undermine the effect of the invention, a vesicle-containing cosmetic can be produced.

The cosmetic according to the present invention may take any form including such as lotion, milky lotion, cream, serum, cleansing, pack, shampoo, conditioner, hair conditioner, bath additive, makeup cosmetic and sunscreen and preferably can be incorporated in lotion.

Since lotions often contain a lower alcohol such as ethanol for dissolving lipid, it has been difficult for a conventional vesicle to maintain stability of lipid membrane in the lotions. On the other hand, a vesicle according to the present invention containing an acyl methyl taurate as a membrane component can maintain high stability even in the lotions containing a lower alcohol such as ethanol.

### EXAMPLE

Vesicles were obtained in accordance with the formulations shown in Table 1 below and the production method and evaluated for the stability thereof. The stability was evaluated by the method below.

### Measurement of turbidity change

Each of the samples immediately after vesicle preparation, and after storage at 0°C, RT (room temperature) and 50°C for one month, was measured for absorbance (Abs.) at a wavelength of 600 nm by an absorptiometer (Ubest-55, JASCO Corporation). The value (Abs. after storage/Abs. immediately after preparation) was obtained and expressed as a turbidity change. The results are shown in Table 2.

### [Appearance

The vesicle dispersion state one hour after preparation of each test sample was visually observed and evaluated based on the following standards. The results are shown in Table 2.
O : aggregate or precipitate is not observed.
Δ : aggregate or precipitate is slightly observed.
X : aggregate or precipitate is significantly observed.

**Table 1**

| | Experimental Example | | | | | | | | |
|---|---|---|---|---|---|---|---|---|---|
| (Component.) | 1 | 2 | 3 | 4 | 5 | 6 | 7 | 8 | 9 |
| (1) Sucrose stearic acid diester (*1) | 2.5 | 2.5 | 2.5 | 2.5 | -- | -- | -- | -- | -- |
| (Diester content: 40%) | | | | | | | | | |
| (2)Sucrose stearic acid diester(*2) | -- | -- | -- | -- | 1 | 1 | 1 | 1 | 1 |
| (99% purity) | | | | | | | | | |
| (3) Dipropylene glycol | 5 | 5 | 5 | 5 | 5 | 5 | 5 | 5 | 5 |
| (4) Arbutin | 3 | 3 | 3 | 3 | 3 | 3 | 3 | 3 | 3 |
| (5)Sodium stearoyl methyl taurate | 0.05 | -- | -- | -- | 0.05 | -- | -- | -- | -- |
| (6)Sodium lauroyl methyl taurate | -- | 0.05 | -- | -- | -- | 0.05 | -- | -- | -- |
| (7)Stearyl trimethylammonium chloride | -- | -- | 0.05 | -- | -- | -- | 0.05 | -- | -- |
| (8)Sodium stearyl sulfate | -- | -- | -- | 0.05 | -- | -- | -- | 0.05 | -- |
| (9) Ion exchanged water | Balance | Balance | Balance | Balance | Balance | Balance | Balance | Balance | Balance |

| | | | | | | | | | |
|---|---|---|---|---|---|---|---|---|---|
| *1: Raw material for sucrose stearic acid diester containing about 40% of sucrose stearic acid diester, and 20 to 23% of a monoester and 33 to 39% of a triester as impurities. *2: Raw material *1 for sucrose stearic acid diester is purified to a purity of 99% by the method according to Japanese Patent No. 3126193. | | | | | | | | | |

### (Production method)

(1) or (2) was dissolved in (3) at 80°C. This was added to a solution mixture of (4), one of (5) to (8) and (9) heated to 80°C and mixed while stirring by a homo mixer (9000 rpm) for 3 minutes and then cooled to obtain a desired sample.

**Table 2**

| | Experimental Example | | | | | | | | |
|---|---|---|---|---|---|---|---|---|---|
| Storage temperature | 1 | 2 | 3 | 4 | 5 | 6 | 7 | 8 | 9 |
| 0°C | 1.002 | 0.998 | 1.004 | 0.999 | 1.056 | 1.130 | 1.012 | 1.005 | 1.035 |
| R T | 1.010 | 1.002 | 1.150 | 1.121 | 1.102 | 1.050 | 1.145 | 1.134 | 1.200 |
| 37°C | 1.100 | 1.105 | 1.153 | 1.204 | 0.699 | 0.998 | 1.135 | 1.145 | 1.213 |
| Appearance | ○ | ○ | Δ | Δ | ○ | ○ | ○ | ○ | Δ |

From the results of Table 2, it was confirmed, in Experimental Examples 1, 2, 5 and 6, that uniform vesicles free from aggregation are formed by incorporating an acyl methyl taurate in a sucrose fatty acid diester. Furthermore, after stored at 37°C, a slight turbidity change was observed; however, turbidity values after storage under other temperatures were the same as that of immediately after preparation. The vesicles are confirmed to be extremely stable.

On the other hand, in Experimental Examples 3, 4, 7 and 8 where other ionic surfactants were added in place of an acyl methyl taurate, large increases of turbidity were observed compared to Experimental Examples 1, 2, 5 and 6. Expansion or collapse of vesicles presumably occurred with time. Particularly, Experimental Examples 3 and 4 where a raw material for sucrose stearic acid diester containing 60% of impurities (monoester, triester) was used, aggregate and precipitation were observed even visually.

Furthermore, also in Experimental Example 9 where no ionic surfactant was added, formation of uniform vesicles was not observed and storage stability based on turbidity was inferior to other Experimental Examples.

From the results above, it was apparent that vesicles excellent in stability can be obtained by incorporating a predetermined amount of acyl methyl taurate in sucrose stearic acid diester. Furthermore, vesicles excellent in stability can be obtained from a raw material for a sucrose fatty acid diester containing 60% of impurities by incorporating an acyl methyl taurate.

### EXAMPLE 2

Cosmetics (lotion) were prepared in accordance with the formulations and the production method shown in Table 3 and vesicles in each of the samples were evaluated for stability. The evaluation method was as follows.

### Measurement of turbidity change

Each of the samples immediately after preparation, and after storage at -5°C, 0°C, RT (room temperature) and 37°C for one month, was measured for absorbance (Abs.) at a wavelength of 600 nm by an absorptiometer (Ubest-55, JASCO Corporation). The value (Abs. after storage/Abs. immediately after preparation) was obtained and expressed as a turbidity change. The results are shown in Table 4.

Furthermore, the samples of Experimental Examples 11 and 12 were stored at - 5°C, 0°C, RT (room temperature) and 37°C for 2 months and turbidity change thereof were obtained in the same manner. The results are shown in Table 5.

**Table 3**

| | Experimental Example | | | |
|---|---|---|---|---|
| (Component) | 10 | 11 | 12 | 13 |
| Sucrose stearic acid diester (*2) | 0.5 | 0.5 | 0.5 | 0.5 |
| (99% purity) | | | | |
| Dipropylene glycol | 5 | 5 | 5 | 5 |
| Dynamite glycerin | 5 | 5 | 5 | 5 |
| Ethanol | 5 | -- | -- | 5 |
| Sodium stearoyl methyl taurate | -- | -- | 0.05 | 0.05 |
| Citric acid (for food) | 0.02 | 0.02 | 0.02 | 0.02 |
| Sodium citrate | 0.08 | 0.08 | 0.08 | 0.08 |
| EDTA3Na · 2H₂O | 0.03 | 0.03 | 0.03 | 0.03 |
| Phenoxyethanol | 0.3 | 0.3 | 0.3 | 0.3 |
| Ion-exchanged water | Balance | Balance | Balance | Balance |
| Abs. immediately after preparation | 0.836 | 0.530 | 0.436 | 0.435 |
| pH | 6.39 | 6.07 | 6.16 | 6.16 |

### (Production method)

Sucrose stearic acid diester was dissolved in dipropylene glycol heated to 80°C. This was added to a solution mixture of the remaining component (water phase) heated to 80°C and mixed/stirred by a homo mixer (9000 rpm) for 3 minutes and then cooled to obtain a desired lotion.

**Table 4**

| | Experimental Example | | | |
|---|---|---|---|---|
| Storage temperature | 10 | 11 | 12 | 13 |
| - 5°C | 1.66 | 1.54 | 1.00 | 1.00 |
| 0°C | 1.42 | 1.46 | 1.00 | 1.00 |
| R T | 1.21 | 1.13 | 0.99 | 1.00 |
| 37 °C | 1.61 | 1.51 | 0.99 | 1.02 |
| Appearance | Δ | Δ | ○ | ○ |

**Table 5**

| Storage temperature | 11 | 12 |
|---|---|---|
| - 5°C | 2.16 | 0.99 |
| 0°C | 2.01 | 1.00 |
| R T | 1.71 | 0.99 |
| 37°C | 2.02 | 1.09 |

As shown in Table 4, compared to Experimental Examples 10 and 11 containing no acyl methyl taurate, Experimental Examples 12 and 13 containing the component were significantly excellent in vesicle formation by appearance and stability with time based on turbidity change. Particularly, when compared to Experimental Examples 10 with 11, turbidity change was significantly large and stability is low in Experimental Example 11 containing ethanol. In contrast, in Experimental Examples 12 and 13, high stability was maintained regardless of presence or absence of an ethanol content.

Furthermore, from Table 5, the sample of Experimental Example 11 stored for 2 months was further lowered in stability; whereas, in Experimental Example 12, the stability of vesicles was maintained.

From the results, it was apparent that a vesicle-containing cosmetic excellent in long-term stability can be obtained by incorporating a predetermined amount of acyl methyl taurate in sucrose stearic acid diester. Furthermore, the vesicle has a high stability even in the lotion containing an alcohol.

Subsequently, lotions containing an alcohol according to the formulations shown in Table 6 below were evaluated for moisturizing effect and usability. Evaluation methods thereof are as follows.

### Moisturizing effect

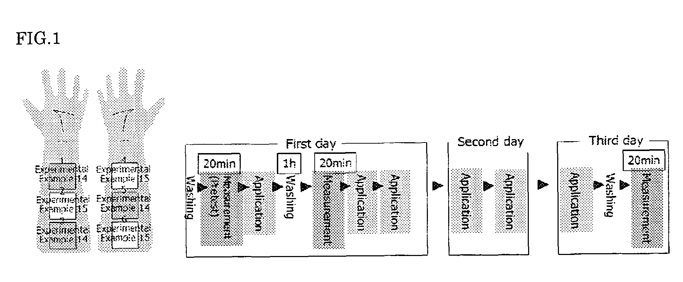

Based on the following test method, a moisturizing effect was evaluated. Note that, the outline of the test is shown in Fig. 1.

The palm side of the forearm of a subject was washed and separated into 5 cm-square test sections. Twenty minutes after washing, the moisture content of the horny layer of each section was measured by an epidermal horny-layer moisture content measuring apparatus (SKICON-200). Thereafter, the sample of each Experimental Example was applied (2ml/cm²) to each section and allowed to leave for one hour. Thereafter, the sample applied was washed away. Twenty minutes later, the horny-layer moisture content was measured again. After the measurement, application of the sample was repeated twice at a time interval.

The next day (second day), application of the sample was repeated twice at a time interval.

Further, the next day (third day), the sample was applied and washed away after one hour. Twenty minutes later, the horny-layer moisture content was measured.

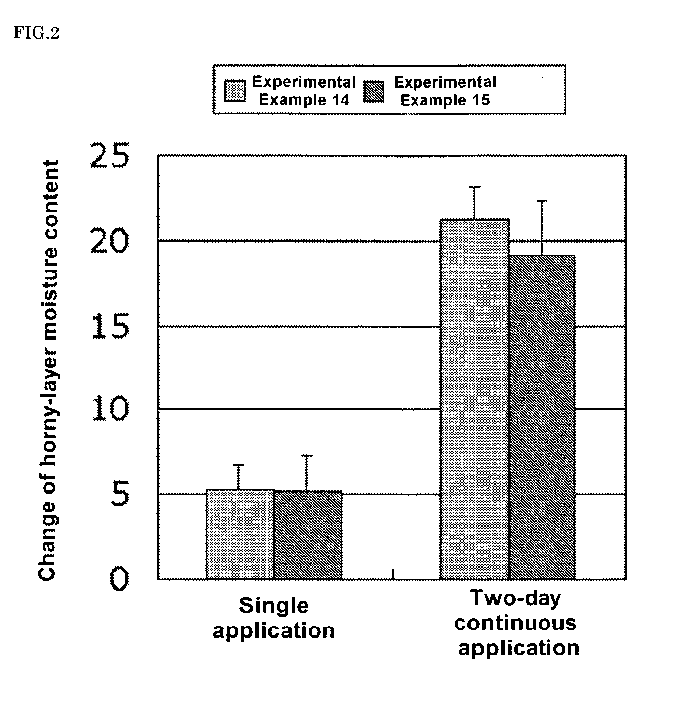

A change of horny-layer moisture content in sections of each Experimental Example is shown in Fig. 2. Note that, the change of horny-layer moisture content represents an increase of the honey layer moisture content based on the measurement value before a sample is applied on the first day. The "single application" indicates an increase measured on the first day, after a sample is first applied. The "two-day continuous application" represents an increase measured on the third day.

### Usability

The samples of Experimental Examples 14 and 15 were subjected to a use test to be checked by nine special panelists and evaluated in accordance with the following evaluation standards.

### <Evaluation of Experimental Example 14 in comparison with Experimental Example 15 (Evaluation of product)>

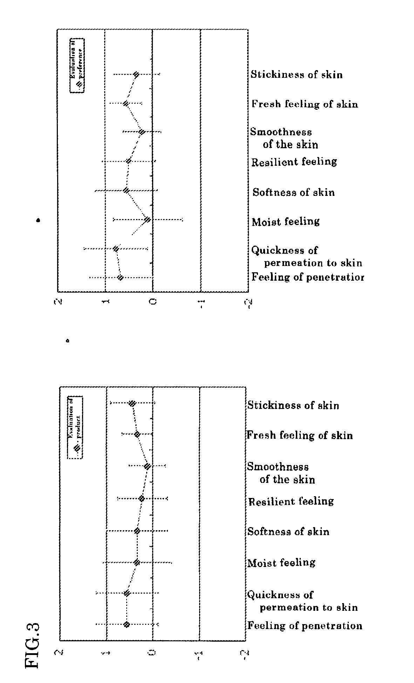

Usability of Experimental Example 15 was regarded as 0. The usability of Experimental Example 14 was evaluated between -2 to 2 in accordance with the following standard and averaged. The average value is shown in the graph of Fig. 3 on the left side

<Separate evaluation on usability of Experimental Example 14 (Evaluation of preference)>

The usability of Experimental Example 14 was separately evaluated between -2 to 2 in accordance with the following standard and averaged.
The average value is shown in the graph of Fig. 3 on the right side.

(Feeling of penetration)
+2: Experimental Example provides feeling of penetration so much during application.
+1: Experimental Example provides feeling of penetration during application.
0: Neither provides feeling of penetration nor provides no feeling of penetration.
-1: Experimental Example hardly provides feeling of penetration during application.
-2: Experimental Example provides feeling of penetration not at all during application.

(Quickness of permeation to skin)
+2: Experimental Example permeates to skin very quickly during application.
+1: Experimental Example permeates to skin quickly during application.
0: Neither permeates to skin quickly nor does not permeate to skin.
-1: Experimental Example hardly permeates during application.
-2: Experimental Example permeates not at all during application.

(Moist feeling)
+2: Experimental Example provides moist feeling so much after application.
+1: Experimental Example provides moist feeling after application.
0: Neither provides moist feeling nor does not provide moist feeling.
-1: Experimental Example hardly provides moist feeling after application.
-2: Experimental Example provides moist feeling not at all after application.

(Softness of skin)
+2: Experimental Example provides softness of the skin so much after application.
+1: Experimental Example provides softness of the skin after application.
0: Neither provides softness of the skin nor does not provide softness of the skin.
-1: Experimental Example hardly provides softness of the skin after application.
-2: Experimental Example provides softness of the skin not at all after application.

(Resilient feeling)
+2: Experimental Example provides resilient feeling so much after application.
+1: Experimental Example provides resilient feeling after application.
0: Neither provides resilient feeling nor does not provide resilient feeling.
-1: Experimental Example hardly provides resilient feeling after application.
-2: Experimental Example provides resilient feeling not at all after application.

(Smoothness of the skin)
+2: Experimental Example provides smoothness of skin so much after application.
+1: Experimental Example provides smoothness of skin after application.
0: Neither provides smoothness of skin nor does not provide smoothness of skin.
-1: Experimental Example hardly provides smoothness of skin after application.
-2: Experimental Example provides smoothness of skin not at all after application.

(Fresh feeling of skin)
+2: Experimental Example provides fresh feeling of skin so much after application.
+1: Experimental Example provides fresh feeling of skin after application.
0: Neither provides fresh feeing of skin nor does not provide fresh feeling of skin.
-1: Experimental Example hardly provides fresh feeling of skin after application.
-2: Experimental Example provides fresh feeling of skin not at all after application.

(Stickiness of skin)
+2: Experimental Example provides stickiness of skin not at all after application.
+1: Experimental Example hardly provides stickiness of skin after application.
0: Neither provides stickiness of skin nor does not provide stickiness of skin.
-1: Experimental Example provides stickiness of skin after application.
-2: Experimental Example provides stickiness of skin so much after application.

**Table 6**

| | Experimental Example | |
|---|---|---|
| (Component) | 14 | 15 |
| Sucrose stearic acid diester | 0.3 | |
| (99% purity) | | |
| Dipropylene glycol | 5 | |
| Dynamite glycerin | 5 | 5 |
| Ethanol | 5 | 5 |
| Sodium stearoyl methyl taurate | 0.03 | - |
| S-safe 1324 (General surfactant) | - | 0.3 |
| Phenoxyethanol | 0 .3 | 0.3 |
| Ion-exchanged water | Balance | Balance |

### (Production method)

Sucrose stearic acid diester was dissolved in dipropylene glycol heated to 80°C. This was added to a solution mixture of the remaining component (water phase) heated to 80°C and mixed/stirred by a homo mixer (9000 rpm) for 3 minutes and then cooled to obtain a desired lotion.

As shown in Fig. 2, the horny-layer moisture content increases in single application. Virtually no difference was observed in amount of increase between Experimental Example 14, in which a vesicle formed of a sucrose fatty acid diester and an acyl methyl taurate was incorporated, and Experimental Example 15, in which a general surfactant was incorporated in place of the vesicle.

When the horny-layer moisture content was measured after application was made two days, an increase of the moisture content was observed in both of Experimental Examples 14 and 15. The moisture content of the application site of Experimental Example 14 was higher.

Furthermore, as shown in Fig. 3, a cosmetic (Experimental Example 14) containing a vesicle formed of a sucrose fatty acid diester as a membrane component exhibited excellent usability in all items compared to a general cosmetic containing a general surfactant.

From the results above, it is apparent that a vesicle obtained by incorporating a predetermined amount of acyl methyl taurate in a sucrose fatty acid diester exhibits a high moisturizing effect and excellent usability when used in a lotion containing an alcohol.

## Claims

1. A vesicle comprising:
a sucrose fatty acid diester and
sodium stearoyl methyl taurate or sodium lauroyl methyl taurate in an amount of 5 to 30 wt% relative to the diester,
wherein the sucrose fatty acid diester is contained as a main component of membrane.

2. The vesicle according to claim 1 wherein a content of impurities in the sucrose fatty acid diester is up to 60%.

3. A cosmetic comprising the vesicle according to claim 1 or 2.

4. A lotion in which the vesicle according to claim 1 or 2 is dispersed in a solvent containing alcohol.

5. A method for producting the vesicle according to claim 1 or 2 comprising:
(I) dissolving a sucrose fatty acid diester in a water-soluble solvent under heating, and
(II) mixing a solution obtained in (I) and an aqueous solvent containing sodium stearoyl methyl taurate or sodium lauroyl methyl taurate.

## Patentansprüche

1. Vesikel, umfassend
- einen Saccharose-Fettsäurediester,
- Natriumstearoylmethyltaurat oder Natriumlauroylmethyltaurat in einer Menge von 5 bis 30 Gewichtsprozent, bezogen auf den Diester,
wobei der Saccharose-Fettsäureester als ein Hauptbestandteil in der Membran enthalten ist.

2. Vesikel nach Anspruch 1, wobei der Gehalt an Verunreinigungen im Saccharose-Fettsäurediester bis zu 60 % beträgt.

3. Kosmetikum, enthaltend das Vesikel nach Anspruch 1 oder 2.

4. Lotion, in der das Vesikel nach Anspruch 1 oder 2 in einem Lösungsmittel dispergiert ist, welches Alkohol enthält.

5. Verfahren zur Erzeugung der Vesikel nach Anspruch 1 oder 2, umfassend
- (I) Lösen eines Saccharose-Fettsäurediesters unter Erwärmen in einem wasserlöslichen Lösungsmittel und
- (II) Vermischen der in (I) erhaltenen Lösung mit einem wässrigen Lösungsmittel, welches Natriumstearoylmethyltaurat oder Natriumlauroylmethyltaurat enthält.

## Revendications

1. Vésicule comprenant :
un diester de sucrose et d'acide gras et
du stéaroyl-méthyl-taurate de sodium ou du lauroyl-méthyl-taurate de sodium dans une quantité de 5 à 30 % en poids par rapport au diester,
dans laquelle le diester de sucrose et d'acide gras est contenu en tant que composant principal de la membrane.

2. Vésicule selon la revendication 1, dans laquelle la teneur en impuretés dans le diester de sucrose et d'acide gras s'élève jusqu'à 60 %.

3. Produit cosmétique comprenant la vésicule selon la revendication 1 ou 2.

4. Lotion dans laquelle la vésicule selon la revendication 1 ou 2 est dispersée dans un solvant contenant de l'alcool.

5. Procédé de production de la vésicule selon la revendication 1 ou 2, comprenant :
(I) une dissolution d'un diester de sucrose et d'acide gras dans un solvant hydrosoluble sous chauffage, et
(II) un mélange d'une solution obtenue en (I) et d'un solvant aqueux contenant du stéaroyl-méthyl-taurate de sodium ou du lauroyl-méthyl-taurate de sodium.
